# EUROPEAN PATENT APPLICATION

(11) **EP 4 624 546 A2**
(43) Date of publication of application: **01.10.2025**
(21) Application number: 25196531.5
(22) Date of filing: 29.04.2022
(51) Int. Cl.: C09J 139/06

(54) **HOT MELT WETNESS INDICATOR ADHESIVE AND PRODUCTION PROCESS THEREOF**

(30) Priority: 30.04.2021 PT 2021117210
(62) Divisional of application: 22725536.1
(71) Applicant: Colquimica-Indústria Nacional de Colas, S.A., 4440-578 Valongo (PT)
(72) Inventor: SOUSA PIMENTA, Ana Isabel, 4580-575 Lordelo (PT); FERNANDES FRUTUOSO, Cristina Isabel, 4450-150 Matosinhos (PT); SILVA COUTINHO, Ana Paula, 4575-032 Alpendurada e Matos (PT)
(74) Representative: Patentree

(57) **Abstract**

The present invention relates to a hot melt wetness indicator adhesive for personal hygiene disposable articles, specifically, to a hot melt that does not cause skin irritation or skin allergies. The hot melt wetness indicator adhesive is comprised of a polymeric component, a resin component, a plasticizer component, a surfactant component, a pH indicator, and an antioxidant component.

## Description

### Field of the invention

The present invention relates to a hot melt wetness indicator adhesive for personal hygiene disposable articles, specifically, to a hot melt that does not cause skin irritation or skin allergies.

### Summary of the invention

Wetness indicators were introduced in the personal hygiene disposable articles industry in 1978. The usage of this technology in personal hygiene disposable articles allowed for the visual identification of contact with liquids, such as body fluids, for example, but not limited to urine, blood, vaginal discharges, breastmilk, sweat or faeces, has occurred. This contact is easily identified by the colour change of the wetness indicator in personal hygiene disposable article.

Currently, the performance of wetness indicators in personal hygiene disposable articles relies on the colour change. Thus, a wetness indicator needs to have in its composition a chromoactive component - a component with the ability to change its colour according to external conditions. By using a chromoactive component, there is an initial colour associated with the absence of wetness and a final colour associated with the presence of wetness.

In the wetness indicators field, the prevailing chromoactive components are hydrochromic inks and pH indicators. Hydrochromic inks are solvatochromic materials, which are materials sensitive to specific solvents, in this case to aqueous solutions such as water. Molecularly, hydrochromic inks have two arrangements, one of them colourless, and the change between them is triggered by the presence or absence of aqueous solutions. On the other hand, pH indicators allow to visually identify the pH of the surrounding environment. There are several pH indicators, each with a colour change that occurs within a defined pH range of values. Thus, the incorporation of a pH indicator in the composition of a wetness indicator assumes that when the wetness indicator is in contact with aqueous solutions, such as water, moisture, wetness or body fluids, the pH will change which will be signalled by a colour transition.

The present invention relates to a hot melt wetness indicator adhesive composition in which the chromoactive component is a pH indicator. The wetness indicator adhesive of the present invention aims to detect the presence of aqueous solutions in personal hygiene disposable articles, namely, but not limited to disposable baby, adult, and incontinence diapers. Thus, the usage of a pH indicator as the chromoactive component of the present invention assumes that the pH of the hot melt wetness indicator adhesive is inferior or superior to the mentioned fluids so the contact between them can be effectively signalled. For reference, the pH of urine is typically comprised between 4,5 and 8,0. Therefore, to properly work, the present invention composition must have a pH inferior to 4,5 or superior to 8,0.

In a preferred embodiment, the present invention relates to a hot melt wetness indicator composition that has an acidic composition in the absence of water, preferably, a pH inferior to 4,5. In another embodiment, the present invention relates to a hot melt wetness indicator composition that has an alkaline composition in the absence of water, preferably, a pH superior to 8,0.

Therefore, in the preferred embodiment, the present invention composition must incorporate acidic raw materials. For this purpose, rosin resins are popular since they are highly acidic and contribute simultaneously to properties that are desired in a hot melt adhesive, such as adhesion and tack.

Rosin resins are extracted from the pine tree and consist in a mixture of several low molecular weight organic acids. In the hot melt adhesives industry, these resins are recognized by the great adhesion capacity and compatibility with a wide range of polymers. For reference, the acid number of a pure rosin resin is typically comprised between 150 and 180 mg KOH/g, while the typical acid number of a hydrocarbon resin is about 0,1 mg KOH/g. Although useful and commonly used in the hot melt adhesives industry, the usage of such resins in personal hygiene disposable articles is not recommended due to its proven harmful character.

There is growing evidence that unmistakably show that rosin resins provoke skin irritation and skin sensitization. Contact dermatitis is the most common adverse reaction reported in literature and can be allergic or irritating. While skin irritation is a local inflammatory response, allergy is an immune response in which the adverse reactions can appear in locals other than the contact zone. Thus, rosin resins are recognized as a substance that not only can cause an allergic reaction to pre sensitized individuals, but also as a substance that can induce an allergic reaction to healthy individuals.

The usage of rosin resins is widespread in everyday products, such as paper, cosmetics, medical devices, gum, detergents, adhesives, and recreational objects, among others. Studies reveal that the occurrence of allergies in individuals that frequently use some of the aforementioned objects is often due to the presence of rosin resin. An example of this was the replacement of rosin resin by other substances in Stradivarius violin bows. The resin in the bow increases the friction between the materials to influence the generated sound, and consequently, converts the resin to dust. After establishing the connection between the allergies in violins users and the resin dust, rosin resins were replaced by another substance.

Even with an extensive knowledge about skin irritation and skin allergies caused by rosin resins, there is no effective treatment for it. Avoiding exposure to rosin resins is the only effective treatment to avoid adverse reactions. On top of this, the risk to develop adverse reactions depends on the contact zone and how extensive it is. Concerning personal hygiene disposable articles, the usage is frequent and constant. Therefore, there is a need and urgency to eliminate substances that are known to be skin irritating or sensitizing from personal hygiene disposable articles, as is the case of rosin resins.

Following the state of the art, the present invention relates to a hot melt wetness indicator adhesive without raw materials that can provoke skin irritation and/or sensitization in its composition, specifically, but not limited to rosin resins. Furthermore, the typical technical properties of a hot melt wetness indicator adhesive must be maintained: fast colour transition and intense colour before and after contact with aqueous solutions, strong resistance to premature indication, strong colour retention and good processability to allow an easy industrial application.

### Background of the invention

Ever since entering the market of personal hygiene, inventions relating to wetness indicators have been issued in an attempt to improve the technical properties thereof.

Document US4743238 describes the composition of a hot melt adhesive in which the polymeric component comprises soluble or water sensitive polymers, being the chromoactive component a pH indicator that changes its colour when in contact with aqueous solutions. The preferred embodiments take advantage of rosin resins to adjust the speed of the colour change and improve colour retention after contact with aqueous solutions.

Following this, the document US5342861 describes a composition that aims to technically improve the previous invention, specifically the thermal stability, compatibility of the composition and the colour intensity after contact with aqueous solutions. Terpene phenolic resins, rosin esters resins or mixtures thereof are preferred components of the invention.

Document WO 2011/071807 describes a wetness indicator suitable for personal hygiene disposable articles with a cellulose free absorbent core technology. This technology is specially demanding for the long term performance of wetness indicators since it has a high content of superabsorbent polymers and surfactants, both extremely alkaline components, which can provoke a premature indication. Rosin resins are one of the preferred components for the composition of this invention.

Document WO 2020/190625 describes an invention relating to a wetness indicator with a halogen free pH indicator in its composition, with a good thermal stability, intense colour and resistance do die out after wetting. Once again, rosin resins are one of the preferred raw materials for the invention composition.

Document WO 2016/161329 describes a wetness indicator with a hydrochromic ink as the chromoactive component and several insoluble polymers, a surfactant, and an acid component, such as rosin resin, in its composition.

### Advantages of the invention

It is evident that the state of the art evolved to keep pace with increasingly demanding personal hygiene market technologies. Nevertheless, the preferred raw materials for hot melt wetness indicator adhesives are still the same, particularly in the case of polymers and acidic components, such as rosin resins. Therefore, it is possible to deduce the difficulty to replace these raw materials while maintaining technical properties and performance.

The present invention relates to a hot melt wetness indicator adhesive with a non-irritating and non-sensitizing composition, achieved by not using raw materials known to cause these adverse reactions, such as rosin resins. In accordance with the state of the art, it is intended that the product of this invention is suitable for the most demanding technologies in personal hygiene disposable articles market, including technologies with a high content of alkaline substances, such as superabsorbent and surfactants.

The selection of all components for the hot melt adhesive of the present invention was made based on the initial selection of the resin component, also known as the tackifier. As referred previously, the state of the art comprises almost exclusively hot melt adhesive compositions which use rosin resins. The use of rosin resins is preferred, due to its high solubility. Tackifiers with a high cloud point, such as hydrocarbon resins, on the other hand, are harder to solubilize.

Due to the allergenic characteristic of rosin resins, the exchange of rosin resins for hydrocarbon resins was always desired. However, no solution for this long felt need was found. Although some documents present embodiments that use hydrocarbon resins, these embodiments were always indicated as having some issue that made them not preferable for commercial or industrial use. The hot melt adhesive composition of the present invention therefore presents the advantage of having solved a gap in the state of the art that had not yet been figured out, being applicable in industrial production lines without causing damages to machines and other industrial components.

### Detailed description of the invention

The present invention relates to a hot melt wetness indicator adhesive capable of signalling the presence of wetness.

The present invention also relates to a hot melt wetness indicator adhesive that changes its colour in the presence of aqueous solutions and with a controlled response time.

The present invention also relates to a hot melt wetness indicator adhesive that changes its colour in the presence of aqueous solutions with an adjustable response time through the hot melt wetness indicator adhesive composition.

The present invention relates to a hot melt wetness indicator adhesive that adheres to one or more substrates and signals immediately once the substrate is wetted.

The present invention, a hot melt wetness indicator adhesive, comprises:
- a polymeric component consisting in water sensitive thermoplastic polymers;
- a resin component consisting in hydrocarbon resins with a cloud point superior to 70 °C;
- a plasticizer component comprising at least one plasticizer capable of producing a homogeneous mixture with the polymeric component and also with the ability to adjust rheological properties, such as viscosity;
- a surfactant component comprising at least one non-ionic surfactant;
- a pH indicator, specifically, a pH indicator able to signal a pH change between 4,5 and 8,0;
- an antioxidant component comprising at least one phenolic antioxidant.

By "aqueous solutions" is meant any solution which presents water as solvent, be it in the liquid or gaseous state, such as, but not exclusively, water, moistness, wetness, humidity and body fluids such as urine, saliva, tears, snot, sweat and blood.

By "substrate" is meant the material or the material surface in which the hot melt wetness indicator adhesive is applied and/or contacts directly with. In the context of personal hygiene disposable articles, typical substrates are polyethylene film and polypropylene non-woven.

By "open time" is meant the maximum period of time after the adhesive is applied in the first substrate in which it is possible to effectively bond a second substrate.

By "setting time" is meant the minimum period of time necessary to compress two or more substrates to achieve a strong bond. Thus, the setting time is related with the recovery speed of the adhesive cohesive strength.

By "affinity for water", also known as hydrophilicity, is meant the ability to establish intermolecular bonds with water molecules. This property, typical of polar molecules, defines the attraction and interaction of different molecules with water molecules.

By "polymeric component" is meant the polymeric materials that contribute to the main properties of the adhesive, considered the backbone of the composition. Therefore, the remaining components of the invention composition are intended to modify or enhance properties of the polymeric component.

By "extremely slow colour change" is meant a colour transition that takes more than 10 minutes to occur or be completed.

By "instant colour change" is meant a colour transition that takes less than 1 minute to occur or be completed.

By "resin compatibility with the remaining composition" is meant the ability to make a homogenous mixture, without phase separation.

Since the invention relates to a hot melt adhesive, properties such as adhesion, cohesion, open time, setting time, among others, should not be overlooked. However, as it is a wetness indicator adhesive, properties as solubility and acidity/pH are imperative since these have a direct impact on the adhesive performance and stability.

The solubility or affinity for water of the hot melt wetness indicator adhesive, also known as remoistenability, is essential in assuring an effective colour change. By being soluble or partially soluble, it is possible for the water to diffuse through the hot melt adhesive and, therefore, contact with the pH indicator that changes its colour. If the hot melt wetness indicator adhesive is insoluble or not soluble enough, the colour change will not occur or will occur very slowly. On the other hand, if the composition of the hot melt wetness indicator adhesive is too soluble, the colour change will be instantaneous and the adhesive will have a poor colour retention, perceived by colour fading, which is not desired. In this context, a colour change that takes until 10 minutes to be completed after contact with aqueous solutions is considered acceptable. Thus, the composition solubility must be carefully fine-tuned to achieve the desired properties.

### Polymeric component

The polymeric component of the hot melt wetness indicator adhesive should contain exclusively water sensitive thermoplastic components, meaning that the polymer should be soluble or partially soluble in water. Suitable water sensitive polymers for the present invention include, namely, but not exclusively, polyvinylpyrrolidone (PVP), polyvinylpyrrolidone/vinyl acetate copolymers (PVP/VA), polyethylene glycol (PEG), polyacrylic acid (PAA), ethylene and acrylic acid copolymer (EAA), poly(vinyl acetate) (PVA) and mixtures thereof.

The polymeric component represents up to 70% by weight of the hot melt wetness indicator adhesive composition, preferably between 5% and 60%, more preferably between 10% and 50%, and even more preferably between 30% and 45%, more concretely between 35 and 40%.

Some of the polymers mentioned are hygroscopic, meaning that they have the ability of absorbing moisture from the surrounding environment. The usage of such polymers can pose an issue since it promotes the occurrence of premature indication. As suggested, premature indication occurs when the hot melt wetness indicator adhesive contacts with alkaline materials, causing a colour change before contacting with aqueous solutions. Another potential risk are the recent technologies used in personal hygiene disposable articles with a high incorporation of alkaline materials, such as superabsorbent polymers, mineral fillers, and surfactants. The hot melt wetness indicator adhesive exposure to the mentioned risks and, therefore, premature indication, occurs frequently during the storage of personal hygiene disposable articles. Considering this, the incorporation of hygroscopic polymers should be minimized to reduce odds of a premature indication.

### Resin component

To be soluble or to have affinity for water, polymers must be polar. For this reason, polar raw materials are generally preferred in the state of the art. By being highly acidic and, therefore, being polar, rosin resins are a popular choice when formulating hot melt wetness indicator adhesives since they contribute simultaneously to the affinity for water and to the acidity of the composition.

Since the hot melt wetness indicator adhesive of the present invention aims to be non-irritating and anti-allergic for the skin, rosin resins and their derivatives will be excluded from the composition. Alternatively, the resin component of the present invention consists in hydrocarbon resins, although this is made more difficult by the considerably lower polarity and absence of acidity.

Additionally, the cloud point was also taken into consideration when selecting the resin component for the wetness indicator adhesive of the present invention. The polarity and the aromaticity of resin components (generally called tackifiers) can be assessed by the cloud point. The cloud point measures the temperature at which the tackifier, diluted in a suitable solvent, turns the mixture opaque while cooling, due to phase separation. As is known in the state of the art, tackifiers which have a low cloud point present greater polarity, as is the case of rosin resins (from -5 °C to 10 °C). The higher the cloud point, the lower the aromatic content and/or polarity of the tackifier. Hydrocarbon resins, due to their low polarity, can present a cloud point range from 40 °C to 100 °C. For the present invention, tackifiers with a high cloud point (<70 °C) were selected.

As such, the resin component for the wetness indicator adhesive of the present invention consists in hydrocarbon resins that can be selected from, but not limited to, aliphatic resins (C5), aromatic resins (C9) and aliphatic-aromatic resins (C5/C9), either partially or fully hydrogenated, and dicyclopentadiene (DCPD), preferably C5 and C9 resins, and more preferably C9 resins due to the presumable higher aromatic content. The polarity of C9 resins depends on the hydrogenation degree: the higher the hydrogenation degree, the lower the polarity of the resin and, presumably, the lower the compatibility with the remaining composition. Consequently, it is expected that the intensity of the final colour should reduce as the hydrogenation level increases.

The resin content should be up to 50% by weight of the hot melt wetness indicator adhesive composition, preferably between 5% and 40%, more preferably between 10% and 35% and even more preferably between 15% and 30%, more concretely between 20 and 25%.

### Plasticizer component

In the present invention, the plasticizer component allows the melting of the polymer, the adjustment of the viscosity and softening point of the hot melt wetness indicator adhesive composition to values that make it easily applied in industrial lines. By "acceptable viscosity" is meant a viscosity value at 120°C comprised between 500 and 15.000 mPa·s, preferably between 750 and 10.000 mPa·s and more preferably between 1.000 and 6.000 mPa·s. By "Acceptable softening point" is meant a value comprised between 50 and 120°C, preferably between 55 and 110 °C, more preferably 60 and 100 °C, even more preferably between 60 and 90 °C, more concretely between 70 and 80 °C. Assuring that the viscosity and softening point of the composition are comprised within the mentioned acceptable values, the working temperature in industrial lines is also assured to be acceptable, specifically, between 70 and 160 °C, preferably between 75 and 140 °C and even more preferably between 80 and 130 °C.

Considering this, a plasticizer component comprising at least one liquid plasticizer at room temperature is preferred. Also, since the need to include acidic raw materials is bigger due to the replacement of rosin resins by hydrocarbon resins, acidic plasticizers are preferred. Suitable plasticizers for the present invention can be selected from, but not exclusively, saturated, or unsaturated fatty acids, particularly dimer fatty acids, polymerized fatty acids or derivatives thereof.

A liquid plasticizer at room temperature is a substance with low molecular weight and, as a result, can easily migrate from the hot melt wetness indicator adhesive net. Thus, the usage of such component and its migration can promote a neutralization reaction between the hot melt wetness indicator adhesive and the surface that is applied on, meaning that premature indication can occur. On top of this, a high content of acidic raw materials can lead to damages in the equipment that is applying the hot melt wetness indicator adhesive. Considering this, the plasticizer content should be enough to plasticize the polymer and adjust the pH of the composition to values that allow a good performance without presenting the aforementioned undesired properties.

The plasticizer content should be up to 70% by weight of the hot melt wetness indicator adhesive, preferably from 5% to 60%, more preferably between 10% and 50% and even more preferably between 30% and 45%, more concretely between 35 and 40%.

### Surfactant component

The surfactant component aims to increase the affinity of the composition for water, meaning that it will contribute to the composition solubility. The surfactant component comprises at least one of the following: non-ionic surfactant, cationic surfactant, anionic surfactant, or amphiphilic surfactant. Also, the hydrophilic-lipophilic balance (HLB) of the surfactant component should be comprised between 3 and 20, preferably between 11 and 20, to assure that it is solubilizing.

Non-ionic surfactants are preferred for the present invention and can be selected from, but not exclusively, natural or synthetic ethoxylated alcohols and ethoxylated phenol derivatives.

The surfactant content should be up to 25% by weight of the composition of the present invention, preferably between 0,5% and 20%, more preferably between 1% and 15% and even more preferably between 2% and 10%, more concretely between 2 and 5%.

### pH indicator

The pH indicator must effectively signal a pH change within a pH range of 4,5 to 8,0 - the typical pH of urine. As mentioned, the present invention relates to a hot melt wetness indicator adhesive that in the absence of water has a pH inferior to 4,5. Considering this, a suitable pH indicator for the present invention can be selected from, but not exclusively, among methyl yellow, bromophenol blue, congo red, methyl red or bromocresol green. Bromophenol blue and bromocresol green are preferred pH indicators for the present invention, more preferably bromocresol green. Bromocresol green is yellow for a pH inferior to 3,8 and blue for pH superior to 5,4. Therefore, by using bromocresol green as a pH indicator, the hot melt wetness indicator adhesive will be yellow in the absence of water. When in contact with aqueous solutions the invention will transition from yellow to green and, finally, to blue.

The pH indicator content should be up to 5% by weight of the presentation invention composition, preferably between 0,05% and 3% and more preferably between 0,1% and 2%, more concretely between 0,1 and 0,5%.

### Antioxidant component

The usage of an antioxidant component aims to avoid the degradation of the hot melt wetness indicator adhesive due to excessive heat in the course of its production process, as well as during the application and storage of the personal hygiene disposable articles that is applied on. A suitable antioxidant component for the present invention comprises at least a primary antioxidant, a secondary antioxidant, or a multifunctional antioxidant. From these, suitable antioxidant components can be selected among phenolic antioxidants, phosphite antioxidants, thioesters-based antioxidants, or mixtures thereof, preferably phenolic.

The antioxidant component content should be up to 15% by weight of the composition of the present invention, preferably between 0,5% and 10% and more preferably between 1% and 5%, more concretely between 1 and 2%.

### Hot melt wetness indicator adhesive production process

To produce the hot melt wetness indicator adhesive of the present invention, the first step is to blend the plasticizer and polymeric components at room temperature, until a homogeneous mixture is achieved. Next, the obtained mixture is heated at a maximum temperature of 135 °C with constant stirring until the polymer is completely melted and the mixture turns translucid. The final step is to add the remaining components: resin, surfactant, pH indicator and the antioxidant components, without a specific order. The temperature and stirring should be constant until all the components of the composition are incorporated, and the mixture is homogeneous.

### Embodiments of the invention

The ageing test aims to accelerate the ageing of the hot melt wetness indicator adhesive to identify in a short period of time if any undesired results occur, specifically premature indication or fading of the hot melt wetness indicator adhesive after wetting. "Poor" ageing indicates that any of the mentioned issues occur, while "good" ageing means none of the mentioned issues occur.

The compatibility test allows to check if the raw materials of the composition are compatible, meaning that no phase separation occurs, i.e. the composition maintains homogeneity. As such, it is clear that a "poor" compatibility indicates phase separation, and a "good" result indicates homogeneity.

The processability, also referred to as machinability refers to the performance of the hot melt adhesive composition while being applied on the substrates. An application made at temperatures between 90 °C and 130 °C, without dripping, clogging or build-up of adhesive in the nozzle and/or rolls is considered "good". The occurrence of said events, on the other hand, is considered as "poor".

The thermal stability test evaluates the adhesive degradation due to heat exposure by placing the adhesive at 150 °C for 90 hours and assess the ageing behaviour. By "poor" thermal stability is meant the formation of gel, skin layer, variations in viscosity or softening point and/or darkening of the adhesive. It is considered a "good" a thermal stability if a darker colour still occurs, however the composition continues to be in a liquid state, none of the mentioned effects occurring.

The embodiment of example 1 relates to a hot melt wetness indicator adhesive composition with technical properties such as viscosity, softening point and colour transition considered acceptable. However, this embodiment includes rosin resin in its composition, presenting a poor resistance to premature indication, as seen by the unacceptable ageing tests. This embodiment is not acceptable for the present invention but provides a comparison for the following embodiments as being the industry standard of hot melt wetness indicator adhesives.

The embodiment represented by Example 2 represents a hot melt wetness indicator adhesive composition with an optimized acidity and solubility, while maintaining technical properties, such as viscosity, softening point and colour change, within the mentioned acceptable values. The improved resistance to premature indication, and the consequent compliance with the ageing tests, reveal the composition optimization.

Example 3 presents a hot melt wetness indicator adhesive with an aliphatic resin. This embodiment produced a hot melt wetness indicator adhesive with a dark green colour, therefore, precluding the desired colour transition.

Examples 4 and 5 explore the range of aromaticity of C9 hydrocarbon resins. Hydrocarbon resin 2 has a cloud point inferior to 20 °C and, thus, promotes the affinity with water. On the other hand, hydrocarbon resin 3 has a cloud point superior to 70 °C, being considerably less polar than the previous.

**Table 1. Example 1 to 5 embodiments regarding the adhesive formulation**

| | 1 | 2 | 3 | 4 | 5 |
|---|---|---|---|---|---|
| Polymeric component | 23,8 g | 36,1 g | 36,1 g | 36,1 g | 36,1 g |
| Rosin resin | 46,8 g | 22,2 g | - | - | - |
| Hydrocarbon resin 1 | - | - | 22,2 g | - | - |
| Hydrocarbon resin 2 | - | - | - | 22,2 g | - |
| Hydrocarbon resin 3 | - | - | - | - | 22,2 g |
| Plasticizer component | 22,0 g | 37,2 g | 37,2 g | 37,2 g | 37,2 g |
| Surfactant component | 6,0 g | 3,0 g | 3,0 g | 3,0 g | 3,0 g |
| Antioxidant component | 1,3 | 1,3 g | 1,3 g | 1,3 g | 1,3 g |
| pH indicator | 0,1 g | 0,2 g | 0,2 g | 0,2 g | 0,2 g |
| Viscosity at 120 °C (mPa·s) | 1.200 | 4.300 | 4.400 | 4.300 | 4.600 |
| Softening Point | 60 °C | 75 °C | 76 °C | 72 °C | 73 °C |
| Initial colour | Yellow | Yellow | Dark green | Yellow | Yellow |
| Colour transition | < 1 minute | < 1 minute | - | < 1 minute | < 1 minute |
| Compatibility | Compatible | Compatible | - | Compatible | Compatible |
| Ageing | Poor | Good | - | Good | Good |
| Machinability | Good | Good | - | Poor | Good |
| Thermal stability | Good | Poor | - | Good | Good |

| | | | | | |
|---|---|---|---|---|---|
| Hydrocarbon resin 1: aliphatic tackifier with a cloud point between 30 °C and 60 °C Hydrocarbon resin 2: aromatic tackifier with a cloud point inferior to 20 °C Hydrocarbon resin 3: aromatic tackifier with a cloud point superior to 70 °C | | | | | |

As can be observed, contrary to what expected, a resin component with a higher cloud point, therefore less polar, improved the characteristics of the hot melt adhesive composition. Such was not obtained with a resin component with a lower cloud point, therefore more polar, which indicates that an unexpected effect was obtained in example 5.

### Embodiments of the invention production process

As mentioned, it is imperative for the hot melt adhesive of the present invention that the composition is acidic. However, the acidic components present a poor thermal stability due to the insaturation of the chemical bonds. Therefore, the production process must minimize the heat exposure.

Examples 6-10 represent several production parameters and their influence on the adhesive performance. While high temperatures and long stirring time lead to a fast colour transition and poor colour retention (Example 6), lower temperatures and stirring time produce a slow colour transition by industrial standards (Example 10). An optimum balance of colour transition, colour retention and thermal stability can be achieved (Example 9) by using mild temperatures and intermediate stirring time.

Examples 11-12 represent several methods for the production process of the wetness indicator adhesive. If the polymeric component, when melted alone, burns, the addition of the plasticizer components speeds the melting. By plasticizing the polymer at room temperature and melting this mixture as the first step of the production process, the total stirring time and the heat exposure are minimized, therefore contributing to a better thermal stability and an acceptable colour transition and retention.

**Table 2. Example 6 to 10 embodiments regarding the production process parameters of the adhesive formulation**

| | Example 6 | Example 7 | Example 8 | Example 9 | Example 10 |
|---|---|---|---|---|---|
| Temperature | 150 °C | 150°C | 135 °C | 135 °C | 135 °C |
| Stirring time | 4h30 | 3h30 | 4h30 | 3h30 | 2h30 |
| Colour transition | Instantaneous | Good | Good | Good | Too slow |
| Colour retention | Considerable fade | Slight fading | Slight fading | Good | Good |
| Thermal stability | Poor | Good | Good | Good | - |

**Table 3. Example 11 to 13 embodiments regarding the aggregation order of the components**

| | Example 11 | Example 12 | Example 13 |
|---|---|---|---|
| 1^{st} step | Melting the polymer, the resin, and the antioxidant components. | Melting the plasticizer, resin, and antioxidant components. | Blending the polymer and the plasticizer components at room temperature until homogeneous. |
| 2^{nd} step | -¹ | Adding the remaining components until homogenous, with constant stirring. | Heating the mixture until the polymer melts, with constant stirring. |
| 3^{rd} step | -¹ | - | Adding the remaining components until homogenous. |
| Temperature | 135 °C | 135 °C | 135 °C |
| Stirring time | -¹ | 4h30 | 3h30 |

| | | | |
|---|---|---|---|
| ¹ Led to the burning of the polymer | | | |

As would be clear to a person skilled in the art, the hot melt adhesive composition of the present invention can additionally comprise any other component that is generally used in the state of the art to improve characteristics of hot melt adhesives, such as, but not exclusively, oils, stabilizers, pigments, dyestuffs, antiblock additives, polymeric additives, defoamers, preservatives, thickeners, rheology modifiers, humectants, fillers, solvents, nucleating agents, chelating agents, gelling agents, processing aids, cross-linking agents, neutralizing agents, flame retardants, fluorescing agents, compatibilizers, antimicrobial agents, and water.

## Claims

1. Hot melt wetness indicator adhesive composition comprising: from 5% to 60% by weight of a polymeric component, from 5% to 60% by weight of a plasticizer component, from 0,5% to 20% by weight of a surfactant, from 0,05% to 3% by weight of a pH indicator, from 0,5% to 10% by weight of an antioxidant component, wherein it further comprises from 5% until 40% by weight of a resin component consisting in aromatic hydrocarbon resins with a cloud point superior to 70 °C; and wherein the hot melt wetness indicator adhesive composition is soluble in aqueous solutions.

2. Hot melt wetness indicator adhesive composition according to the previous claim, wherein the aromatic hydrocarbon resin consists in a partially hydrogenated aromatic C9 hydrocarbon resin.

3. Hot melt wetness indicator adhesive composition according to the previous claim 1, wherein the aromatic hydrocarbon resin consists in a fully hydrogenated aromatic C9 hydrocarbon resin.

4. Hot melt wetness indicator adhesive composition according to any of the previous claims, wherein the colour of the adhesive transitions from yellow to blue in the presence of water or aqueous solutions due to a pH indicator being selected from bromophenol blue or bromocresol green.

5. Hot melt wetness indicator adhesive composition according to any of the previous claims, wherein the polymeric component consists in water sensitive thermoplastic polymers selected from polyvinylpyrrolidone (PVP), polyvinylpyrrolidone/vinyl acetate copolymers (PVP/VA), polyethylene glycol (PEG), polyacrylic acid (PAA), ethylene and acrylic acid copolymer (EAA), poly(vinyl acetate) (PVA).

6. Hot melt wetness indicator adhesive composition according to any of the previous claims, wherein the plasticizer component comprises at least one liquid plasticizer at room temperature selected from saturated or unsaturated fatty acids, dimer or polymerized fatty acids or fatty acids derivatives.

7. Hot melt wetness indicator adhesive composition according to any of the previous claims **characterized by** the surfactant component consisting in a non-ionic surfactant with a hydrophilic-lipophilic balance (HLB) comprised between 3 and 20.

8. Hot melt wetness indicator adhesive composition according to any of the previous claims wherein the antioxidant component is selected from phenolic antioxidants, phosphite antioxidants, thioesters-based antioxidants, and mixtures thereof.

9. Hot melt wetness indicator adhesive composition according to any of the previous claims wherein the viscosity at 120°C is comprised between 1.000 and 6.000 mPa·s.

10. Hot melt wetness indicator adhesive composition according to any of the previous claims wherein the softening point is comprised between 60 and 90 °C.

11. Hot melt wetness indicator adhesive composition according to any of the previous claims wherein the weight of a resin component ranges between 10% and 35%; preferably 15% and 30%, more preferably 20 and 25%.

12. Personal hygiene disposable article comprising the hot melt wetness indicator adhesive composition according to any of the previous claims.

13. Personal hygiene disposable article according to the previous claim, wherein the article is disposable baby diaper, adult diaper, or incontinence diaper.

14. Use the hot melt adhesive composition according to any of the previous claims as a wetness indicator, wherein said adhesive composition does not cause skin irritation or skin allergies.

15. Production process of the hot melt wetness indicator adhesive composition according to any of the previous claims comprising the following steps:
a. blending the plasticizer and polymeric components at room temperature until a homogeneous mixture is obtained;
b. heating the prepared mixture until 135 °C, with constant stirring, until the polymeric component melts and becomes translucid;
c. next, the remaining components are added without a specific order; wherein the remaining components are resin, surfactant, pH indicator and the antioxidant components;
d. maintaining the heating and stirring until complete incorporation of all the components, being the total stirring time equal to 3 hours and 30 minutes.
